# EUROPEAN PATENT APPLICATION

(11) **EP 4 092 134 A1**
(43) Date of publication of application: **23.11.2022**
(21) Application number: 20913084.8
(22) Date of filing: 16.01.2020
(51) Int. Cl.: C12Q 1/6837

(54) **METHOD AND SYSTEM FOR DNA DETECTION**

(71) Applicant: Hitachi High-Tech Corporation, Minato-ku Tokyo 105-6409 (JP)
(72) Inventor: TANAKA, Junko, Tokyo 100-8280 (JP); NAKAGAWA, Tatsuo, Tokyo 100-8280 (JP); SHIRATORI, Akiko, Tokyo 100-8280 (JP); UEMATSU Chihiro, Tokyo 100-8280 (JP)
(74) Representative: MERH-IP Matias Erny Reichl Hoffmann Patentanwälte PartG mbB
(86) International application number: PCT/JP2020/001171
(87) International publication number: WO 2021/144903

(57) **Abstract**

Provided are a method and a system for accurately distinguishing a micropartition in which a target gene or target DNA is placed and a micropartition in which a pseudogene is placed by a measuring device and accurately counting the target gene or target DNA in digital PCR using melting curve analysis. The method includes the steps of: placing a DNA solution in each of a plurality of partitions; performing PCR; changing a temperature of each of the partitions and measuring a fluorescence intensity; calculating a melting temperature of a double strand DNA for each of the partitions; counting the number of partitions for each type of the DNA; outputting the number of partitions counted for each type of the DNA; and discriminating the first gene and the pseudogene based on the melting temperature and the number of the counted partitions.

## Description

### Technical Field

The present invention relates to a DNA detection method and a DNA detection system, and particularly relates to digital PCR.

### Background Art

Conventional genetic test includes techniques such as PCR (PTLs 2 to 4) and real-time PCR (NPL 1). In these techniques, there is a problem that measurement reproducibility is deteriorated when the amount of a gene to be detected (herein referred to as a "target gene or target DNA") is small.

As a technique for solving this problem, digital PCR (PTL 1) has been developed. Using digital PCR, it is possible to quantify a trace amount of DNA by determining whether DNA is 0 (absent) or 1 (present) using a limitedly diluted sample and detecting the DNA.

An example of a detection method of digital PCR is shown below. First, a DNA polymerase, a primer and a fluorescent-labeled probe necessary for PCR are added to the limitedly diluted specimen to prepare a PCR reaction solution. The PCR reaction solution is divided into micropartitions such as wells or droplets. At this time, one molecule of the target gene or target DNA is contained or not contained in each partition.

Next, the target gene or target DNA in the micropartition is amplified by PCR. The target gene or target DNA can be quantified by measuring a fluorescence intensity of each micropartition after PCR and counting the number of micropartitions having a fluorescence intensity exceeding threshold.

In such digital PCR, since a limitedly diluted specimen is used, it is possible to suppress the influence of a component derived from the specimen, which is a factor that inhibits reaction of PCR. In addition, since a calibration curve is not required, the absolute amount of target DNA can be directly measured.

By the way, in conventional PCR, it is known that reaction efficiency decreases due to the presence of a reaction inhibitor in a reaction solution, formation of a secondary structure of a template DNA, insufficient design of a primer, and the like.

On the other hand, in digital PCR, since the measurement is performed at the end point of the reaction, the reaction efficiency itself of PCR has not been considered to greatly affect the measurement result. However, in practice, even when the measurement is performed at the end point, fluorescence intensity variation due to inhomogeneity of PCR reaction efficiency of each micropartition is large, and measurement reproducibility and measurement accuracy of digital PCR are deteriorated.

Therefore, in order to improve measurement reproducibility and measurement accuracy of digital PCR, the present inventors have developed a technology capable of discriminating a target gene or target DNA in a micropartition by measuring a melting temperature (Tm) of a PCR amplicon even when the PCR reaction efficiency of each micropartition is non-uniform (PTL 5). Specifically, for example, by measuring melting temperature (Tm) at which the target gene or target DNA amplified in the micropartition and the fluorescent-labeled probe dissociate after PCR, a genotype of the target gene or target DNA can be identified by a difference in melting temperature even if the reaction efficiency of PCR is non-uniform.

In the detection of target gene or target DNA, the presence of pseudogene may be problematic. In the process of evolution of organisms, new genes are acquired, while pseudogenes that have lost their functions have also been generated. A pseudogene is one in which a gene has made copies in the process of evolution, one has an original function, the other has changed, and the changed gene has lost its function without acquiring a new function. For this reason, sequences of KRAS gene and KRASP1 gene which is its pseudogene have extremely high homology.

Therefore, when there is a pseudogene in a target gene or target DNA, the target gene or target DNA and the pseudogene cannot be discriminated from each other, and the pseudogene is erroneously counted as the target gene or target DNA, and the copy number of target genes or target DNAs may be larger than the true value.

In the conventional quantitative PCR, the target gene or target DNA and the pseudogene exist in the same one partition. Thus, if a primer is designed by selecting a portion in which the sequence of the target gene or target DNA and the pseudogene does not match even only several bases, amplification of the target gene or target DNA and that of the pseudogene compete, amplification of the pseudogene is suppressed, and the target gene or target DNA can be preferentially amplified.

### Citation List

### Patent Literatures

PTL 1: JP 2013-521764 A
PTL 2: US 4,683,195 A
PTL 3: US 4,683,202 A
PTL 4: US 4,800,159 A
PTL 5: JP 2018-108063 A

### Non-Patent Literature

NPL 1: Genome Res., 10, pp 986-994, 1996

### Summary of Invention

### Technical Problem

However, digital PCR has had a problem that it is difficult to distinguish the pseudogenes.

In digital PCR, unlike quantitative PCR, a reaction solution to which a specimen is added is divided into minute partitions so that one molecule of a target gene or target DNA is contained or not contained in one partition. For this reason, only one of the target gene or target DNA and the pseudogene is present in one partition.

Therefore, even when there are several bases in the pseudogene in a portion that does not match a primer region designed for the target gene or target DNA, there is no competition with the target gene or target DNA in a micropartition containing only the pseudogene, and thus there has been a problem that the pseudogene is amplified and the pseudogene becomes a false positive of the target gene or target DNA.

Distinguishing a target gene or target DNA from a pseudogene is important in reducing false positives and false negatives and improving measurement reproducibility and measurement accuracy.

Therefore, an object of the present invention is to provide a new DNA detection method and a DNA detection system for accurately distinguishing a micropartition in which a target gene or target DNA is placed and a micropartition in which a pseudogene is placed by a measuring device and accurately counting the target gene or target DNA in digital PCR using melting curve analysis.

### Solution to Problem

The present inventors have found that in digital PCR using melting curve analysis, if probes are designed so that the melting temperatures of a target gene or target DNA and a pseudogene are different from each other, the target gene or target DNA and the pseudogene can be discriminated as different genes depending on the melting temperature, and further, it can be determined that they are the target gene or target DNA and the pseudogene from the correspondence relation between the count numbers of two genes, thereby completing the present invention.

An example of a DNA detection method according to the present invention includes the steps of: placing a DNA solution in each of a plurality of partitions, wherein the DNA solution may contain a plurality of types of DNAs including a first gene and a pseudogene of the first gene, and the DNA solution contains a fluorescent-labeled probe or a DNA intercalator; performing PCR in each of the partitions; changing a temperature of each of the partitions during the PCR or after the PCR, and measuring a fluorescence intensity changing with the temperature change for each of the partitions; calculating a melting temperature of a double strand DNA placed in the partition, for each of the partitions, based on a change in the fluorescence intensity accompanying the temperature change; discriminating the type of DNA for each of the partitions based on the melting temperature, and counting the number of partitions for each type of the DNA; outputting the number of partitions counted for each type of the DNA; and discriminating the first gene and the pseudogene based on the melting temperature and the number of the counted partitions.

An example of a DNA detection system according to the present invention includes: an imaging device that captures an image of a device capable of arranging a DNA solution in each of a plurality of partitions, wherein the DNA solution may contain a plurality of types of DNAs including a first gene and a pseudogene of the first gene, and the DNA solution contains a fluorescent-labeled probe or a DNA intercalator; a temperature adjustment unit that changes a temperature of each of the partitions in order to perform PCR in each of the partitions; a database that stores information indicating a reference melting temperature for each of a first gene and a pseudogene of the first gene; an imaging control unit that causes the imaging device to capture an image in order to acquire a fluorescence intensity changing with a temperature change for each of the partitions; a melting temperature memory that stores information indicating a melting temperature of a double strand DNA placed in the partition, for each of the partitions, wherein the melting temperature is obtained based on a change in the fluorescence intensity accompanying a temperature change in an image captured by the imaging device; and an analysis unit that counts the number of partitions in which the first gene is placed and the number of partitions in which the pseudogene is placed using the database and the melting temperature memory, wherein the analysis unit outputs the number of partitions counted for each type of the DNA and discriminates the first gene and the pseudogene based on the melting temperature and the number of counted partitions.

### Advantageous Effects of Invention

According to the present invention, there are provided a new DNA detection method and a new DNA detection system that more accurately distinguish a micropartition in which a target gene or target DNA is placed and a micropartition in which a pseudogene is placed by a measuring device and more accurately count the target gene or target DNA in digital PCR using melting curve analysis.

### Brief Description of Drawings

[FIG. 1] FIG. 1 is an example of a measurement result according to the principle of the DNA detection method according to Embodiment 1.
[FIG. 2] FIG. 2 is an example of a measurement result of digital PCR for detecting wild-type and a mutant of the target gene or target DNA by measuring fluorescence intensities of two different fluorescent dyes.
[FIG. 3] FIG. 3 is a schematic view of a fluorescence measurement unit in Embodiment 1.
[FIG. 4] FIG. 4 is a schematic diagram of a digital PCR system in Embodiment 1.
[FIG. 5] FIG. 5 is an example of a database used in the DNA detection method in Embodiment 1.
[FIG. 6] FIG. 6 is a schematic diagram showing a method for measuring melting temperature of DNA using a fluorescent-labeled probe in the DNA detection method in Embodiment 1.
[FIG. 7] FIG. 7 is an example of data regarding a fluorescence intensity of a fluorescent dye contained in a well accompanying temperature change in Embodiment 1.
[FIG. 8] FIG. 8 is an example of data regarding a melting temperature obtained from a change in the fluorescence intensity of each well accompanying temperature change in Embodiment 1.
[FIG. 9] FIG. 9 is an example showing results of counting genes in partitions in Embodiment 1.
[FIG. 10] FIG. 10 is a flowchart showing one embodiment of a method for performing melting temperature measurement using the devices of FIGS. 3 and 4.
[FIG. 11] FIG. 11 is an example of a measurement result displayed on a monitor.
[FIG. 12] FIG. 12 is an example of a measurement result according to Embodiment 1.

### Description of Embodiments

Objects, features, advantages, and ideas thereof of the present invention will be apparent to those skilled in the art from the description of the present specification. From the description of the present specification, those skilled in the art can easily reproduce the present invention. The embodiments, specific examples and the like of the invention described below illustrate preferred embodiments of the present invention and are shown for purposes of illustration or description and do not limit the present invention. It will be apparent to those skilled in the art that various alterations and modifications can be made based on the description herein without departing from the spirit and scope of the present invention disclosed herein.

### [Embodiment 1]

### (1) Principle and effect of DNA detection method

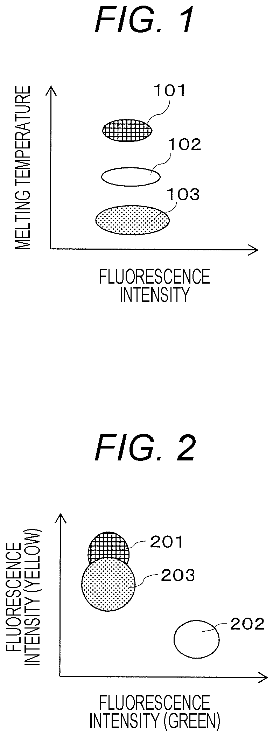

FIG. 1 shows an example of a measurement result according to the principle of the DNA detection method according to Embodiment 1. This example is assumed in a representative embodiment of a method for detecting wild-type of the target gene or target DNA, a mutant of the target gene or target DNA, and a pseudogene of the target gene or target DNA, by calculating melting temperatures of double strand DNAs based on fluorescence intensity changes accompanying temperature changes, for PCR amplicons.

The relationship between the fluorescence intensity and the melting temperature is plotted for a micropartition 101 containing wild-type alleles of the target gene or target DNA, a micropartition 102 containing mutant alleles of the target gene or target DNA, and a micropartition 103 containing pseudogenes of the target gene or target DNA.

In addition, FIG. 2 shows an example of a measurement result of digital PCR for detecting wild-type and a mutant of the target gene or target DNA by measuring fluorescence intensities of two different fluorescent dyes.

In digital PCR, a plurality of types of mutations may be detected by one set of measurements using a fluorescent-labeled probe having different colors for respective mutations (genotype) of DNA. The example of FIG. 2 is a diagram schematically showing results when a yellow fluorescent-labeled probe is used for the wild-type allele of the target gene or target DNA and a green fluorescent-labeled probe is used for the mutant allele of the target gene or target DNA. The relationship between the green fluorescence intensity and the yellow fluorescence intensity is plotted for a micropartition 201 containing wild-type alleles of the target gene or target DNA, a micropartition 202 containing mutant alleles of the target gene or target DNA, and a micropartition 203 containing pseudogenes of the target gene or target DNA.

Here, it is assumed that the fluorescent-labeled probe is complementary to the sequence positioned between a primer pair used in PCR, and is configured such that the probe is degraded and the fluorescent label emits fluorescence when the primer extends. Specifically, a TaqMan (registered trademark) probe can be given as an example.

When the DNA is amplified during PCR, in the micropartition 201 containing wild-type alleles of the target gene or target DNA, the yellow fluorescent-labeled probe corresponding to the wild-type allele of the target gene or target DNA is degraded, and yellow fluorescence is emitted. In addition, in the micropartition 202 containing mutant alleles of the target gene or target DNA, the green fluorescent-labeled probe corresponding to a mutant A allele of the target gene or target DNA is degraded, and green fluorescence is emitted. Neither green nor yellow fluorescence is detected in empty droplets containing no target gene or target DNA (not shown).

However, in practice, as shown in FIG. 2, there may be a pseudogene having a very similar sequence in the target gene or target DNA. Even when there are several bases in the pseudogene in a portion that does not match a primer region designed for the target gene or target DNA, the pseudogene is amplified in the micropartition 203 containing pseudogenes because there is no competition with the target gene or target DNA. As a result, the fluorescent-labeled probe of the target gene or target DNA is degraded to emit yellow fluorescence, and the distribution may overlap with the micropartition 201 containing wild-type alleles of the target gene or target DNA.

In digital PCR using melting curve analysis, it is possible to discriminate between genotypes by utilizing the fact that the melting temperatures of a fluorescent-labeled probe and DNA vary depending on the genotype. The example of FIG. 1 is a diagram schematically showing results of measuring the melting temperatures of DNA in micropartitions using fluorescent-labeled probes corresponding to the wild-type, mutant, and pseudogene respectively for the target gene or target DNA.

Here, as the fluorescent-labeled probe, for example, a well-known molecular beacon can be used. Hereinafter, a DNA detection method will be described in detail using a molecular beacon as an example. A molecular beacon is configured as an oligonucleotide, and has a sequence complementary to a sequence between a primer pair used in PCR for amplifying a target gene or target DNA. In addition, the molecular beacon has mutually complementary sequences at both ends, and a fluorescent dye is provided at one end and a quenching dye (quencher) is provided at the other end.

When a molecular beacon is hybridized with a target gene or target DNA, it emits fluorescence with the fluorescent dye and the quenching dye at both ends being separated from each other. However, when the molecular beacon is dissociated from the target gene or target DNA with an increase in temperature, complementary sequences at both ends are hybridized with each other to form a stem-loop structure, and the fluorescent dye and the quenching dye approach each other to quench the fluorescent dye.

In the micropartition 101 containing wild-type alleles of the target gene or target DNA, a fluorescent-labeled probe corresponding to the wild-type allele of the target gene or target DNA hybridizes to DNA amplified by PCR to emit fluorescence, and a melting temperature corresponding to the fluorescent-labeled probe of the wild-type allele is observed.

In addition, in the micropartition 102 containing mutant alleles of the target gene or target DNA, a fluorescent-labeled probe corresponding to the mutant allele of the target gene or target DNA hybridizes to DNA amplified by PCR to emit fluorescence, and a melting temperature corresponding to the fluorescent-labeled probe of the mutant allele is observed.

Furthermore, in the micropartition 103 containing pseudogenes of the target gene or target DNA, a fluorescent-labeled probe corresponding to the wild-type allele of the target gene or target DNA hybridizes to DNA amplified by PCR in a form containing a mismatch base to emit fluorescence, and a melting temperature corresponding to this mismatch hybridization is observed.

Thus, the presence or absence of a target gene or target DNA having a wild-type allele, the presence or absence of a target gene or target DNA having a mutant allele, and the presence or absence of a target gene or target DNA having a pseudogene can be determined based on the fluorescence intensity, the type (for example, color) of fluorescence, and the melting temperature.

Since the melting temperature of DNA is not affected by the reaction efficiency of PCR, in-plane measurement variation at the time of fluorescence measurement, and the like, it is possible to discriminate the genotype of DNA in a micropartition with high accuracy. For example, the sequence of the fluorescent-labeled probe is determined so that the melting temperature (Tm) of each fluorescent-labeled probe with respect to the target gene or target DNA is different, the fluorescence intensity change accompanying the temperature change is measured with respect to the DNA in the micropartition, the melting curve analysis is performed, and the melting temperature is compared, whereby the genotype of the DNA in the micropartition can be discriminated.

Furthermore, the relationship (for example, ratio) between the copy number of target genes or target DNAs and the copy number of their pseudogenes is determined by the position of each gene on the chromosome, and for example, when both exist on autosomes, their copy numbers are equal to each other. Therefore, the genotype of DNA in each micropartition is counted, and whether or not the relationship between the sum of the wild-type copy number and the mutant copy number of target genes or target DNAs and the copy number of pseudogenes conforms to an expected relationship (for example, 1 : 1) is confirmed, whereby more accurate gene detection becomes possible.

### (2) Configuration example of DNA detection device

The DNA detection system according to Embodiment 1 includes a DNA detection device for detecting a target gene or target DNA in a DNA solution, and executes the DNA detection method described in the present specification. In the present embodiment, the single DNA detection device constitutes the DNA detection system, but a plurality of devices can be designed to cooperate to constitute the DNA detection system.

A DNA detection device includes a temperature adjustment unit for heating a DNA solution, a fluorescence measurement unit for measuring an intensity of fluorescence emitted from the DNA solution, a computer for calculating a melting temperature of a double strand DNA based on a melting curve representing a change in the intensity of the fluorescence accompanying a temperature change of the DNA solution, and a monitor for displaying information transmitted from the computer.

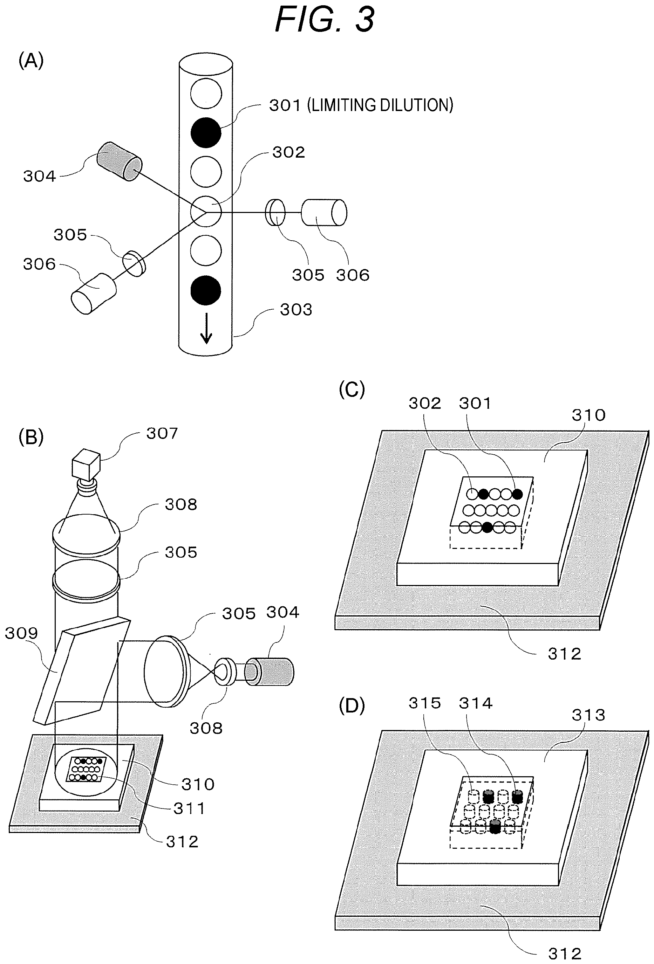

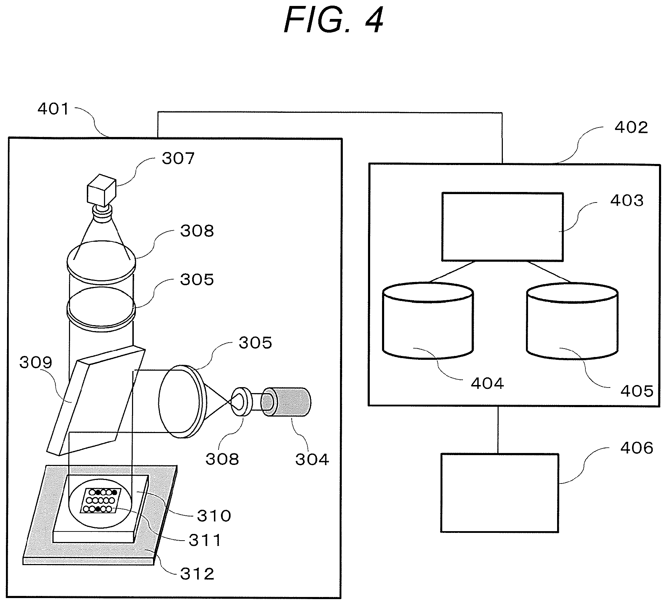

The DNA solution may be in any carrier, for example, may be provided as droplets in oil, or placed in a well such as a plate. As an example of the DNA detection device, a DNA detection device having a fluorescence measurement unit is shown with reference to FIGS. 3 and 4.

FIG. 3 is a schematic view of a fluorescence measurement unit in Embodiment 1, and FIG. 4 is a schematic diagram of a digital PCR system in Embodiment 1. The fluorescence measurement unit of FIG. 3 measures the color and fluorescence intensity of the fluorescent dye contained in a limitedly diluted droplet or well. The digital PCR system of FIG. 4 includes a fluorescence measurement unit exemplified in FIG. 3, a computer that analyzes measurement data, and a monitor that displays a result, and uses melting curve analysis. In these examples, the fluorescence measurement unit measures the color and fluorescence intensity of the fluorescent dye contained in the DNA solution in the droplet or well, but the configuration of the DNA detection device according to the present invention is not limited thereto.

In the example of the fluorescence measurement unit shown in FIG. 3A, the fluorescence intensity of a droplet is measured using a microchannel. A droplet 301 flows in the direction of the arrow in a microchannel 303. When the droplet flows to a measurement position (the position of a droplet 302 in FIG. 3A), the droplet is heated by the temperature adjustment unit (not shown), and a light source 304 irradiates the droplet with excitation light. The fluorescent substance contained in the droplet is excited by the light source 304, and the emitted fluorescence is detected by a photomultiplemeter 306 through a fluorescence filter 305. The photomultiplemeter 306 is an example of an imaging device that captures an image of a device capable of placing a DNA solution in each of a plurality of partitions.

The fluorescence measurement unit includes the light source 304, the fluorescence filter 305, and the photomultiplemeter 306. The fluorescence measurement unit may be separately provided for each color of the fluorescent dye, or may be configured to excite two types of fluorescent dyes with excitation light of one light source and simultaneously detect each fluorescence by two fluorescence filters as shown in FIG. 3A.

In addition, the droplets may be arranged on a plane as shown in FIGS. 3B and 3C, and the color and fluorescence intensity of the fluorescent dye of each droplet may be measured.

This will be described more specifically. For example, a plurality of droplets 311 are arranged in an array in a droplet detection cartridge 310 and set on a temperature control stage 312 which is a temperature adjustment unit. The temperature control stage 312 changes the temperature of each partition in order to perform PCR in each partition. The temperature of the droplet detection cartridge is changed by the temperature control stage 312, and the change in the fluorescence intensity of the droplets accompanying the temperature change is measured. The fluorescence intensity changes differently between the droplet 301 containing the target gene or target DNA and the droplet 302 not containing the target gene or target DNA.

The measurement procedure is as follows, for example. First, each droplet 311 is irradiated with excitation light from the light source 304 through a lens 308, the fluorescence filter 305, and a dichroic mirror 309. The fluorescent substance contained in each droplet 311 is excited by the excitation light, and emitted fluorescence is detected by a CCD camera 307 through the dichroic mirror 309, the fluorescence filter 305, and the lens 308. The CCD camera 307 is an example of an imaging device.

In FIG. 3A, it is necessary to process droplets one by one, but the devices of FIGS. 3B and 3C are preferable in that a large number of droplets can be processed at a time. In addition, the devices of FIGS. 3B and 3C are more suitable than that of FIG. 3A in that the temperature control stage 312 can also be used for DNA amplification reaction.

Further, as shown in FIG. 3D, wells arranged in an array may be used instead of the droplets. The specimen is added so that 1 or 0 target gene or target DNA is contained in one well, and PCR is performed in the well to measure the color and fluorescence intensity of the fluorescent dye of the well.

This will be described more specifically. For example, a reaction solution containing a specimen is added to wells provided in a cartridge 313 for well type detection. Thereafter, PCR is performed in the wells, and the wells are set on the temperature control stage 312 as a temperature adjustment unit. The temperature of the cartridge 313 is changed by the temperature control stage 312, and the change in the fluorescence intensity of the wells accompanying the temperature change is measured. The fluorescence intensity changes differently between a well 314 containing the target gene or target DNA and a well 315 not containing the target gene or target DNA.

The measurement procedure is as follows, for example. First, each well is irradiated with excitation light from the light source 304 through the lens 308, the fluorescence filter 305, and the dichroic mirror 309. The fluorescent substance contained in the reaction solution in the wells is excited by the excitation light, and emitted fluorescence is detected by the CCD camera 307 through the dichroic mirror 309, the fluorescence filter 305, and the lens 308. When the well is used as shown in FIG. 3D, PCR to melting curve analysis can be performed in the cartridge 313 without arranging droplets in the droplet detection cartridge.

When the change in the fluorescence intensity of the well accompanying the temperature change is observed, an inclination adjustment unit (not shown) may be provided under the temperature control stage 312. The inclination adjustment unit removes air bubbles generated in the cartridge 313 by heating by the temperature control stage 312. This prevents that a fluorescence image cannot be acquired due to air bubbles when the fluorescence intensity of each well is measured while the temperature of a sample is lowered by the temperature control stage 312.

As shown in FIG. 4, the fluorescence data detected by a fluorescence measurement unit 401 is sent to a computer 402. In the computer 402, the melting temperature of an amplicon is calculated by an analysis unit 403 and stored in a memory 405. In this manner, the memory 405 functions as a melting temperature memory, and stores, for each partition, information indicating a melting temperature of the double strand DNA placed in the partition. Note that, as described later, the melting temperature is obtained on the basis of a fluorescence intensity change accompanying the temperature change in an image captured by an imaging device (for example, photomultiplemeter 306).

In addition, the relationship among the type, melting temperature, and number of genes is prepared in advance in database 404. In particular, the database 404 stores information indicating a predetermined melting temperature to be standard (reference melting temperature) for each of a target gene or target DNA (first gene) and a pseudogene of the target gene or target DNA. With reference to the database 404, the genotype of the target gene or target DNA is discriminated based on the measured value of the melting temperature of the memory 405, and is counted for each genotype.

The count result is displayed on the monitor 406. The monitor 406 is an example of an output device, and display processing on the monitor 406 may be replaced with output processing to another output device (printing device, non-volatile storage device, or the like).

The DNA detection device according to Embodiment 1 may include a sample dividing unit. The sample dividing unit divides the DNA solution containing the target gene or target DNA into micropartitions. The micropartitions may be configured as wells arranged in an array in a cartridge, or may be configured as droplets dispersed in oil. By configuring the partitions in this way, limiting dilution can be performed appropriately.

In addition, the DNA detection device according to Embodiment 1 may include an amplification unit for amplifying DNA with respect to a micropartition.

### (3) Example of method for analyzing melting curve

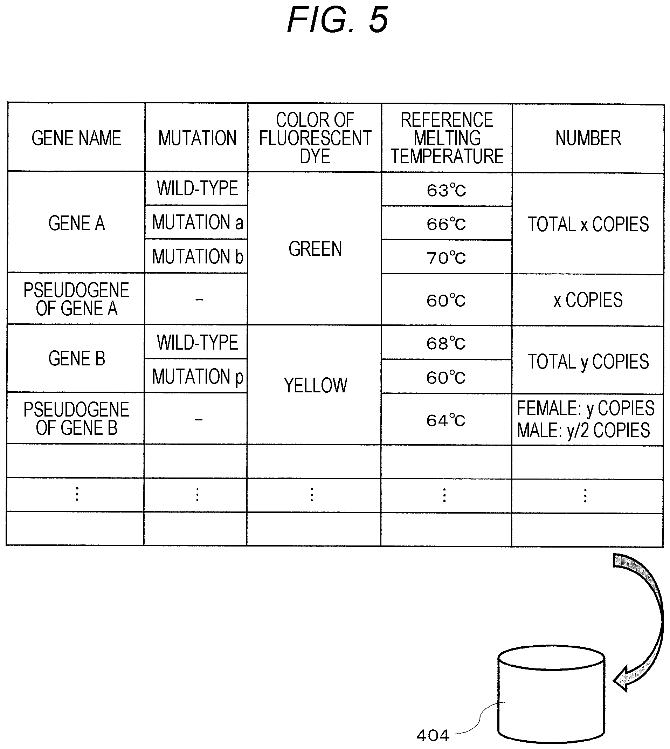

FIG. 5 shows an example of a database that stores information indicating a reference melting temperature of a gene, prepared prior to measurement of digital PCR. The data shown in FIG. 5 can be measured in advance by a pilot experiment or the like and stored in the database 404.

In the example of FIG. 5, for each gene, the reference melting temperature is stored for each of the gene and the pseudogene of the gene. Also, in this example, the reference melting temperature is stored for each genotype. A plurality of mutant may be defined for one gene.

The information indicating a reference melting temperature is defined in this example as a value indicating a single temperature, but may also be defined as information indicating a range of temperatures.

Further, in this example, in addition to the reference melting temperature, information indicating the color of the fluorescent dye is stored for each genotype of each gene.

Furthermore, in this example, for each gene, information indicating the relationship between the number of target genes or target DNAs (genotypes are not distinguished) and the number of their pseudogenes is stored. This relationship is expressed, for example, as a ratio. In the example of FIG. 5, for gene A, when x copies of target genes or target DNAs exist, it is shown that x copies of pseudogenes exist, that is, the ratio of the number of pseudogenes to the number of target genes or target DNAs is 1.

Since the relationship between the copy numbers of target genes or target DNAs and their pseudogenes is determined by their positions on chromosomes, the copy number relationship may be defined for each positional relationship on the chromosome. Specific examples are shown below.

First, a case where the target gene or target DNA is on an autosome will be described. In this case, when the pseudogene is on an autosome, the number of pseudogenes is equal to the number of target genes or target DNAs (gene A in FIG. 5 may be the case). When the pseudogene is on an X chromosome, the number of pseudogenes is equal to the number of target genes or target DNAs in a female specimen, whereas the number of pseudogenes is half the number of target genes or target DNAs in a male specimen (gene B in FIG. 5 corresponds to the case). When the pseudogene is on a Y chromosome, the number of pseudogenes is zero in a female specimen, and the number of pseudogenes is half the number of target genes or target DNAs in a male specimen.

Next, a case where the target gene or target DNA is on an X chromosome will be described. In this case, when the pseudogene is present on an autosome, the number of pseudogenes is equal to the number of target genes or target DNAs in a female specimen, whereas the number of pseudogenes is twice the number of target genes or target DNAs in a male specimen. When the pseudogene is on an X chromosome, the number of pseudogenes is equal to the number of target genes or target DNAs (gene A in FIG. 5 could correspond to the case). When the pseudogene is on a Y chromosome, the number of pseudogenes is zero in a female specimen, and the number of pseudogenes is equal to the number of target genes or target DNAs in a male specimen.

Furthermore, a case where the target gene or target DNA is on a Y chromosome will be described. In this case, the number of target genes or target DNAs is zero in a female specimen. When a male specimen has a pseudogene on an autosome, the number of pseudogenes is twice the number of target genes or target DNAs. When a male specimen has a pseudogene on an X chromosome, the number of pseudogenes is equal to the number of target genes or target DNAs. When a male specimen has a pseudogene on a Y chromosome, the number of pseudogenes is equal to that of target genes or target DNAs.

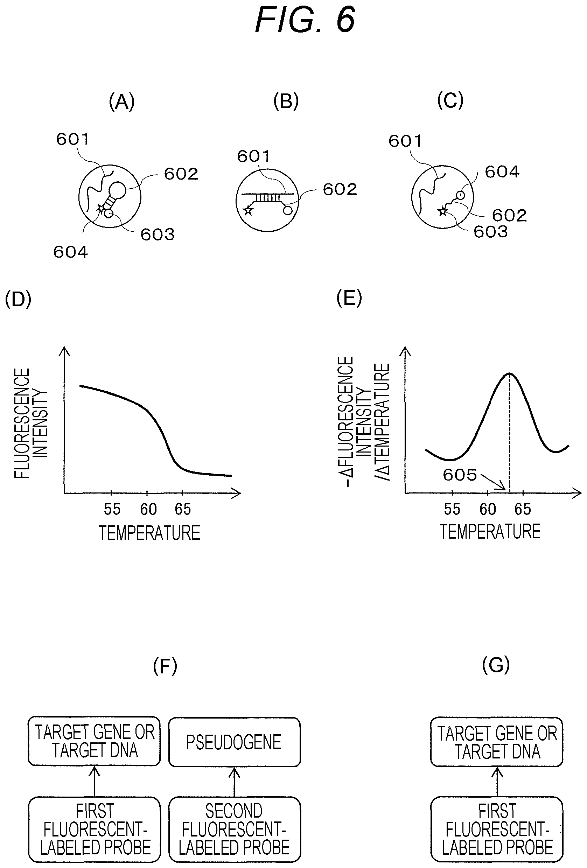

FIG. 6 is a schematic diagram showing a principle that the melting temperature of a target gene or target DNA and a pseudogene can be measured using a fluorescent-labeled probe. As the fluorescent-labeled probe, it is preferable to use a molecular beacon designed to have a structure capable of hybridizing to a target gene or target DNA.

When a fluorescent-labeled probe 602 exists alone in isolation as shown in FIG. 6A, a stem-loop is formed, and a fluorescent dye 603 and a quencher 604 are close to each other, so that fluorescence is not emitted. When the fluorescent-labeled probe 602 is added to the specimen solution in which PCR reaction has been completed, a loop portion of the fluorescent-labeled probe 602 is annealed to DNA 601 amplified in the specimen solution as shown in FIG. 6B at a temperature of about room temperature. As a result, the fluorescent dye 603 and the quencher 604 are separated, so that the fluorescent-labeled probe 602 emits strong fluorescence.

Thereafter, when the specimen solution is heated, the DNA 601 and the fluorescent-labeled probe 602 are dissociated as shown in FIG. 6C, and a stem-loop is formed again in the fluorescent-labeled probe 602 as shown in FIG. 6A, so that the fluorescence intensity from the fluorescent-labeled probe 602 decreases.

FIG. 6D shows an example of results of plotting a melting curve (a curve representing a change in the fluorescence intensity with respect to temperature change) at this time on a graph. Note that this fluorescent-labeled probe may be used in common with a fluorescent-labeled probe for PCR, but a probe different from the fluorescent-labeled probe for PCR may be prepared and used.

Moreover, the measurement of the melting curve may be performed in parallel with the PCR, or may be performed by raising the temperature of the specimen solution independently of the PCR (for example, after completion of the PCR).

In the melting curve of FIG. 6D, a differential curve of a result of differentiating the fluorescence intensity with respect to the temperature (or a result of dividing the fluorescence intensity change by the temperature change) is shown in FIG. 6E. In FIG. 6E, the sign is inverted. The temperature corresponding to an inflection point of the fluorescence intensity is calculated as a melting temperature 605 of double strand DNA. The temperature at the inflection point of the fluorescence intensity in FIG. 6D corresponds to the temperature at the maximum value of the differential curve in FIG. 6E.

As described above, the melting temperature can be calculated as the inflection point of the fluorescence intensity. According to such a calculation method, the melting temperature can be accurately calculated.

The melting temperature of the fluorescent-labeled probe for detecting the target gene or target DNA can be controlled based on a known technique. For example, it can be controlled by changing the sequence or chain length of the probe. Alternatively, it can also be controlled by using artificial DNA such as peptide nucleic acid (PNA) or locked nucleic acid (LNA).

In the fluorescent-labeled probe 602 used here, the combination of the fluorescent dye 603 and the quencher 604 is not particularly limited as long as it is a combination generally used for real-time PCR. Examples of the fluorescent dye 603 include FAM, VIC, ROX, Cy3, Cy5 and the like, and examples of the quencher 604 include TAMRA, BHQ1, BHQ2, BHQ3 and the like.

As the sequence of the fluorescent-labeled probe 602, sequences specific to wild-type of the target gene or target DNA, a mutant of the target gene or target DNA, and a pseudogene of the target gene or target DNA are each prepared, and these may be individually detected. In this case, as shown in FIG. 6F, it can be said that the fluorescent-labeled probe 602 contains a first fluorescent-labeled probe having a sequence corresponding to the target gene or target DNA and a second fluorescent-labeled probe having a sequence corresponding to the pseudogene. In such a configuration, the target gene or target DNA and the pseudogene can be more easily distinguished.

Alternatively, specific sequences are prepared for the wild-type of the target gene or target DNA and the mutant of the target gene or target DNA, and a fluorescent-labeled probe having a sequence specific to the wild-type of the target gene or target DNA may be mishybridized with respect to the pseudogene to detect the target gene or target DNA. In this case, as shown in FIG. 6G, it can be said that the fluorescent-labeled probe 602 contains a first fluorescent-labeled probe having a sequence corresponding to the target gene or target DNA. In such a configuration, the number of fluorescent-labeled probes to be prepared can be reduced.

In a modification of Embodiment 1, a DNA intercalator can be used instead of the fluorescent-labeled probe. As a specific method, first, a DNA intercalator is added to a PCR reaction solution to prepare a specimen solution, and PCR such as PCR is performed. At a temperature of about room temperature, the DNA intercalator binds to a double-stranded DNA amplified in the specimen solution and emits strong fluorescence.

Thereafter, as the temperature of the specimen solution increases, the double-stranded DNA in the specimen solution is dissociated to become single-stranded DNA, and the DNA intercalator does not bind, so that the fluorescence intensity decreases. Incidentally, the measurement of the fluorescence intensity change with respect to the temperature change may be performed by raising the temperature of the specimen solution independently of the PCR (for example, after completion of the PCR).

The melting temperature of the target gene or target DNA can be controlled depending on the sequence and chain length of the sequence of the PCR amplicon by changing the design of the primer.

As the DNA intercalator, an intercalator that increases the fluorescence intensity by binding to double-stranded DNA and can be used for detecting double-stranded DNA is used. Specifically, SYBR (registered trademark) Green I, SYBR Gold, PicoGreen (registered trademark), SYTO (registered trademark) Blue, SYTO Green, SYTO Orange, SYTO Red, POPO (registered trademark)-1, BOBO (registered trademark)-1, YOYO (registered trademark)-1, TOTO (registered trademark)-1, JOJO (registered trademark)-1, POPO-3, LOLO (registered trademark)-1, BOBO-3, YOYO-3, TOTO-3, PO-Pro (registered trademark)-1, YO-Pro (registered trademark)-1, TO-Pro (registered trademark)-1, JO-Pro (registered trademark)-1, PO-Pro-3, YO-Pro-3, TO-Pro-3, TO-Pro-5, ethidium bromide and the like can be applied. When the DNA intercalator is heat resistant, the DNA intercalator can be added to the well or droplet before the PCR reaction is performed.

A method for analyzing the melting temperature of the target gene or target DNA and the pseudogene using the principle of FIG. 6 is shown below.

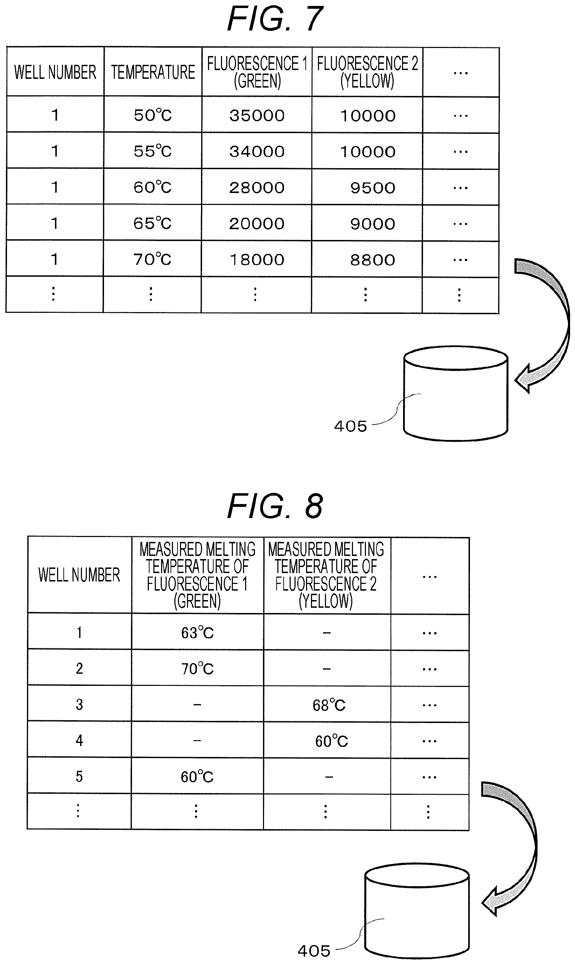

A PCR reaction solution containing a specimen, a fluorescent-labeled probe and enzymes is divided into micropartitions. After the PCR processing, the fluorescence intensity of each partition is calculated based on the fluorescence image acquired while changing the temperature, and stored in the memory 405 as shown in FIG. 7. FIG. 7 shows an example of data stored in the memory in Embodiment 1. This data is acquired, for example, by analyzing the fluorescence intensity of the fluorescent dye accompanying the temperature change in the analysis unit for each well.

In the example of FIG. 7, the fluorescence intensity is measured for a plurality of colors. In this way, discrimination with higher accuracy can be performed. However, the fluorescence intensity may be measured for a single color.

Next, a melting curve of each partition is created based on the fluorescence intensity change with respect to the temperature change of each partition. Next, the value of the melting temperature of each partition is calculated based on the melting curve of each partition, and stored in the memory 405 as shown in FIG. 8. Hereinafter, the melting temperature calculated based on the measurement of the fluorescence intensity as described above is referred to as "measured melting temperature", and is distinguished from a predefined reference melting temperature.

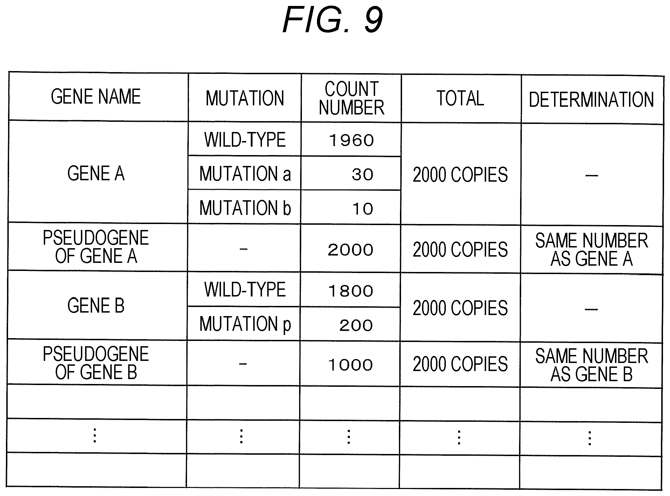

FIG. 9 is an example showing results of counting genes in partitions. As shown in FIG. 9, it is determined whether the gene placed in each partition is the wild-type of the target gene, the mutant of the target gene or target DNA, the pseudogene of the target gene or target DNA, or none of them in the database 404. Next, the number of wild-type of the target gene or target DNA (the number of partitions in which the wild-type of the target gene is placed), the number of mutants of the target gene or target DNA (the number of partitions in which the mutant of the target gene or target DNA is placed), and the number of pseudogenes of the target gene or target DNA (the number of partitions in which the pseudogenes of the target gene or target DNA are placed) are counted.

Next, determination is made on the relationship between the number of target genes or target DNAs (the total number of wild-type and mutants) and the number of pseudogenes with reference to the database 404. The determination is made, for example, based on whether or not the measured ratio of the number of pseudogenes to the number of target genes or target DNAs conforms to the ratio recorded in the database 404.

As a specific example, in the case of detecting gene A shown in FIG. 5, it is determined that there is conformity when the measured ratio of the number of pseudogenes to the number of target genes or target DNAs is 1, and it is determined that there is non-conformity when the measured ratio is not 1. Note that the determination of conformity may be made using a range. For example, in the case of detecting gene A shown in FIG. 5, it may be determined that there is conformity when the measured ratio is within a range of 0.8 to 1.2, and it may be determined that there is non-conformity when the measured ratio is not within the range.

In the case of gene A in FIG. 5, the ratio is 1 or about 1, but the ratio varies depending on the gene, and is any one of 1/2, 1, 2, and 0.

If the measured ratio conforms to the ratio recorded in the database 404, information indicating conformity is displayed on the monitor 406. If there is non-conformity, information indicating non-conformity (for example, information indicating that a measurement error has occurred) is displayed on the monitor 406. These pieces of information can be reflected in accuracy control of digital PCR. In addition, when a measurement error occurs at a frequency equal to or higher than a preset reference, an alert may be displayed on the monitor 406. The alert includes, for example, information indicating that a user needs to adjust a digital PCR system.

### (4) Method for measuring melting temperature

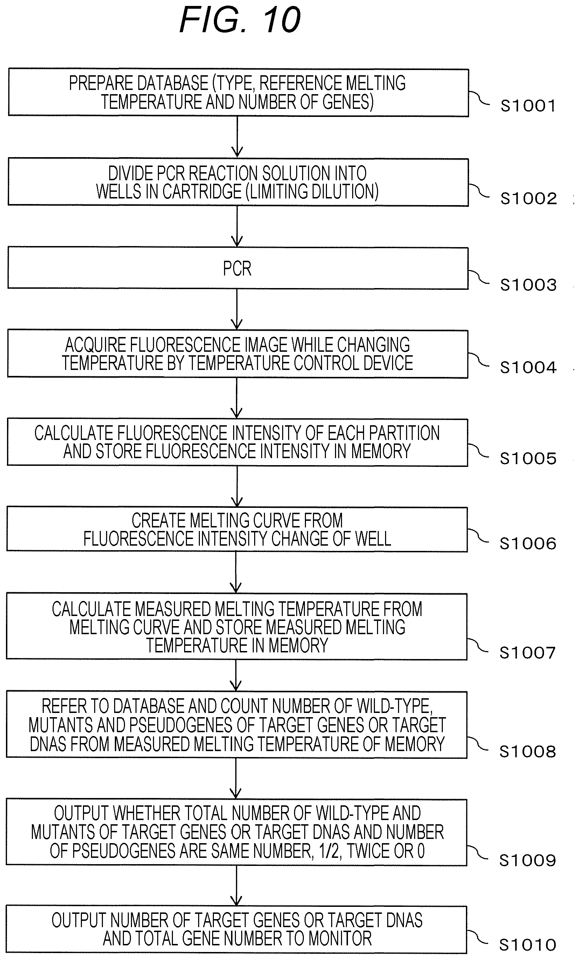

An example of a method for measuring melting temperature will be described with reference to a flowchart of FIG. 10. This example is one embodiment of a method for performing melting temperature measurement using the devices shown in FIGS. 3 and 4 and a cartridge. In this example, a device of FIG 3B, a cartridge with wells of FIG 3D, and a DNA intercalator or molecular beacon are used.

First, a database 404 regarding a gene to be measured by digital PCR is prepared (S1001). Next, a specimen solution derived from a biological sample containing DNA (DNA solution) is prepared. The specimen solution may contain a plurality of types of DNAs including a target gene or target DNA (first gene) and its pseudogene. This specimen solution is added to a PCR reaction solution. The PCR reaction solution contains a DNA polymerase, a primer, a DNA intercalator or a molecular beacon, deoxyribonucleotides, and a buffer solution. As a result, the specimen solution contains a fluorescent-labeled probe or the DNA intercalator.

The PCR reaction solution is divided into wells arranged in an array in a cartridge 313 (S1002). This step is a step of placing the specimen solution in each of the plurality of partitions. Here, in the present embodiment, digital PCR processing is performed. That is, the specimen solution placed in each partition is subjected to limiting dilution, which enables execution of digital PCR.

Next, the cartridge 313 is set in a thermal cycler, and PCR is performed by temperature control of the thermal cycler (S1003). This is a step of performing PCR in each partition. DNA is amplified by repeating a cycle including a denaturation step, an extension step, and an annealing step.

If a DNA intercalator is used, it intercalates to the amplified DNA, and if a molecular beacon is used, it hybridizes to the amplified DNA. This increases the fluorescence intensity. The reaction conditions including the temperature, time, number of cycles and the like of each step can be easily set by those skilled in the art. After PCR, when the temperature is lowered to room temperature, the DNA forms a double strand.

After PCR, a fluorescence image is acquired (S1004) A detailed procedure example is as follows. The cartridge 313 is placed on a temperature control stage 312 of the DNA detection device. While the temperature of the cartridge 313 is changed by the temperature control stage 312, a fluorescence measurement unit 401 measures the fluorescence intensity from the DNA intercalator or molecular beacon of each well. As a result, a fluorescence image is acquired.

Step of S1004 is a step of changing the temperature of each partition and measuring the fluorescence intensity changing with the temperature change for each partition. The step of S1004 may be performed during the PCR or may be performed after the PCR.

In the step of S1004, a computer 402 or another component may function as an imaging control unit. The imaging control unit causes an imaging device (for example, photomultiplemeter 306) to capture an image in order to acquire a fluorescence intensity changing with a temperature change for each partition.

Next, the obtained fluorescence image is sent to the computer 402, and the fluorescence intensity of each micropartition is calculated by an analysis unit 403 and stored in a memory 405 (S1005).

The analysis unit 403 creates a melting curve based on the fluorescence intensity data (S1006), calculates a melting temperature using the melting curve, and stores the melting temperature in the memory 405 (S1007). This step includes a step of calculating, for each partition, the melting temperature of the double strand DNA placed in the partition, based on the fluorescence intensity change accompanying the temperature change. In this way, the measured melting temperature is obtained.

In addition, the analysis unit 403 refers to the data on the reference melting temperature in the database 404, and counts the number of each of wild-type, mutants, and pseudogenes of the target gene or target DNA on the basis of the measured melting temperature of the memory 405 (S1008). Step of S1008 includes a step of discriminating the type of DNA for each partition based on the reference melting temperature and the measured melting temperature, and counting the number of partitions for each type of DNA.

As described above, in S1008, the analysis unit 403 counts the number of partitions in which the target gene or target DNA (first gene) is placed and the number of partitions in which the pseudogene is placed using the database 404 and the memory 405. In addition, in S1008, the analysis unit 403 may determine whether or not the target gene or target DNA and the pseudogene of the target gene or target DNA are contained in the specimen solution.

The distinction between wild-type, mutant, and pseudogene can be made based on the reference melting temperature and the measured melting temperature. For example, in the case of gene A in FIG. 5, if the measured melting temperature is 63°C, it can be determined that it is the wild-type of the target gene or target DNA.

This determination may be made based on a range. For example, when the measured melting temperature according to a well is within a predetermined range (for example, within the reference melting temperature ±1°C) including the reference melting temperature recorded in the database 404 for a genotype, it can be determined that a gene of the genotype is placed in the well. When using range with width in this manner, a robust determination can be made appropriately considering allowable range.

Next, the analysis unit 403 determines the relationship between the number of target genes or target DNAs (the total number of wild-type and mutants) and the number of pseudogenes. That is, the target gene or target DNA and the pseudogene are discriminated from the melting temperature and the number of counted partitions. For example, it is determined whether the ratio is 1, 1/2, 2, 0, or none of these. The result is output (S1009). Here, as described above, this determination may be made based on a range. For example, in step of S1009, the analysis unit 403 determines whether the ratio of the number of partitions in which the pseudogene is placed to the number of partitions in which the target gene or target DNA (first gene) is placed is a value within a range corresponding to 1, a value within a range corresponding to 1/2, a value within a range corresponding to 2, a value within a range corresponding to 0, or a value not within any range. In this way, by automatically determining the correspondence relation, a user of the device can more easily grasp the content of the specimen solution.

Finally, the monitor 406 outputs the number of target genes or target DNAs and the total number of genes in the cartridge to the monitor (S1010). For example, the numbers of partitions counted for respective types of DNA are output.

In S1008, in the determination of whether the DNA of each well is positive (that is, the gene is placed in the well) or negative (that is, the gene is not placed in the well), information on the fluorescence intensity may be used.

For example, a fluorescence intensity value itself can be used as the fluorescence intensity information. If the fluorescence intensity of a well is within a predetermined range, the well is determined to be positive; otherwise the well is determined to be negative.

Alternatively, as the information of the fluorescence intensity, a ratio or a difference of fluorescence intensities at different temperatures may be used. For example, the fluorescence intensity can be standardized by using a ratio or a difference between the fluorescence intensity at a temperature lower than the reference melting temperature and the fluorescence intensity at a temperature higher than the reference melting temperature. For example, if the ratio or difference is within a predetermined range for a well, the well is determined to be positive; otherwise the well is determined to be negative.

For example, by subtracting the fluorescence intensity at 85°C from the fluorescence intensity at 50°C, the fluorescence influence of the fluorescent-labeled probe itself, that is, the background influence, can be removed.

The method for determining the range of fluorescence intensity, the range of reference melting temperature, and the range of the correspondence relation (for example, ratio) between the copy number of target genes or target DNAs and the copy number of pseudogenes can be arbitrarily selected. For example, a pilot experiment or the like may be performed in advance and an operator may statistically determine the ranges from the result, or the DNA detection system may automatically determine the ranges. In addition, for each digital PCR measurement, the measurement data of each well in the cartridge may be used to statistically determine the threshold of the fluorescence intensity and the predetermined range of the reference melting temperature.

The data for statistically discriminating DNA in the well may include any or all of the following items, or may include other items.
- Fluorescence intensity at a temperature lower than the reference melting temperature
- Fluorescence intensity at a temperature higher than the reference melting temperature
- Ratio of the fluorescence intensity at a temperature lower than the reference melting temperature to the fluorescence intensity at a temperature higher than the reference melting temperature
- Difference between the fluorescence intensity at a temperature lower than the reference melting temperature and the fluorescence intensity at a temperature higher than the reference melting temperature
- Characteristic amount representing the reference melting temperature
- Characteristic amount representing the shape of melting curve

The specimen solution to be used is not particularly limited as long as it is a sample containing DNA to be detected, and examples thereof include biological samples (body fluids and tissues, cells, excretions and the like of animals and plants) and soil samples (including fungi, bacteria, and the like).

Examples of the body fluid include blood, saliva, cerebrospinal fluid, and the like. The blood contains cell free DNA (cfDNA), circular tumor DNA (ctDNA), and the like. Examples of the tissue include affected parts (for example, cancer tissues of breast, liver and the like) of a disease obtained by a surgical operation or a biopsy. The tissue may be an already fixed tissue, for example, a formalinfixed paraffin-embedded tissue (FFPE). Examples of the cell include cells (those in an affected part or the vicinity thereof) collected by a biopsy method and circular tumor cells circulating in blood, and the like.

Pretreatment of these specimens is not particularly limited, and those collected from a living body, an environment or the like and then added to a suspension to be homogenized or dissolved in a solution may be used as they are, but it is preferable to use those obtained by extracting or purifying the nucleic acid contained therein.

It is desirable to add oil to the upper surface of a PCR reaction solution so that the PCR reaction solution divided into wells does not evaporate during measurement of PCR and melting curve analysis. The oil is preferably a chemically inert substance that is insoluble or poorly soluble in the PCR reaction liquid, and is preferably a substance that is stable to a temperature change at a high temperature such as PCR. A fluorine-based oil, a silicone-based oil, a hydrocarbon-based oil or the like can be used.

Examples of the fluorine-based oil include perfluorocarbon, hydrofluoroether, and the like. The longer the carbon chain of the fluorine-based oil is, the lower the volatility of the fluorine-based oil is, which is preferable. Examples of the silicone-based oil include polyphenylmethylsiloxane, trimethylsiloxysilicate, and the like. Examples of the hydrocarbon-based oil include mineral oil, liquid paraffin, hexadecane, and the like.

The oil may be used by adding a surfactant. Here, the type of surfactant is not particularly limited, but Tween 20, Tween 80, Span80, Triton X-100 and the like can be applied.

### (5) Display of results

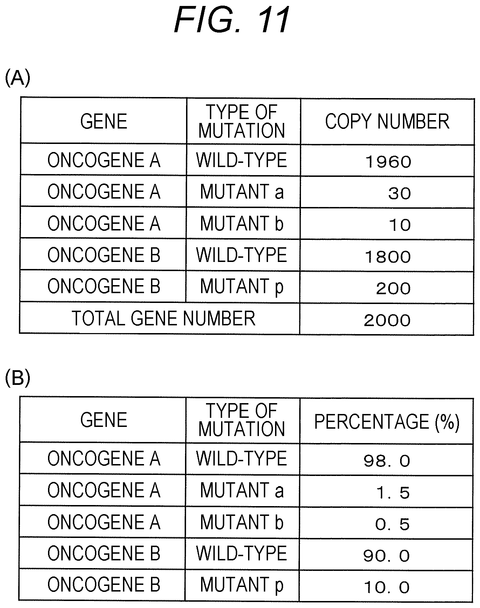

FIG. 11 is an example of a measurement result displayed on the monitor. This example shows results of measuring two types of Oncogenes A and B as target genes or target DNAs. In this example, the same number of Oncogenes A and B are detected.

As shown in FIG. 11A, a number of counted DNAs contained in the specimen solution may be displayed for each type of oncogene and each type of mutation, or as shown in FIG. 11B, a percentage of counted DNAs contained in the specimen solution may be displayed for each type of oncogene and each type of mutation. In the example of FIG. 11B, the percentage of mutant genes in the target gene or target DNA is displayed.

As shown in FIG. 11A, the total gene number of oncogenes may be displayed (in this example, the same number of Oncogenes A and B are detected). The total gene number may be calculated from the sum of the wild-type and mutants of the target gene or target DNA, may be a value obtained by correcting the value with the measurement result of the pseudogene of the target gene or target DNA, or may be a value calculated from the number of target genes or target DNAs or the number of pseudogenes. When such display is performed, the user of the device can easily grasp the content of the specimen solution.

The result displayed on the monitor may be the number or percentage of the specimen solution DNA as shown in FIG. 11, or may be a graph obtained by plotting measured values of the specimen solution on two axes of the fluorescence intensity and the measured melting temperature of the fluorescent-labeled probe as shown in FIG. 1, or may include both of them. In addition, a histogram obtained by plotting the number of DNAs in the specimen solution with respect to the fluorescence intensity of the fluorescent-labeled probe or the measured melting temperature may be included.

The range related to the fluorescence intensity of the fluorescent-labeled probe used for counting the number of DNAs may be arbitrarily changed by the user. In addition, the user may arbitrarily change the range related to the reference melting temperature used when counting the number of DNAs. The DNA detection system may accept an operation for changing these and change the corresponding range. In this way, the user can view the graph or histogram of the measurement result, change the range of the fluorescence intensity and/or the reference melting temperature, and count the number of DNAs in the specimen solution within the new range again.

In addition to the number or percentage of target genes or target DNAs, the count number of pseudogenes may be displayed. Moreover, the DNA detection device may determine whether or not the correspondence relation between the count number of target genes or target DNAs and the count number of pseudogenes is appropriate, and display the result on the monitor 406. For example, it may be determined whether or not the ratio of the count number of pseudogenes to the count number of target genes or target DNAs conforms to the value (or range) stored in the database 404, and if it conforms, information indicating that these count numbers conform (for example, a message indicating that the measurement has been performed normally) may be displayed on the monitor 406, and if not, information indicating that these count numbers do not conform (for example, a message indicating that a measurement error has occurred) may be displayed on the monitor 406.

As described above, since the specimen solution is treated as a solution in a well or a droplet, it may be expressed as the number of wells or the number of droplets instead of the number of DNAs in the specimen solution.

### (6) Program

Another embodiment of the present invention is a program for causing a DNA detection device to perform a DNA detection method. Here, the DNA detection device may be, for example, the DNA detection device according to Embodiment 1, and executes the method described in detail in the above section (1) as the DNA detection method using the device described in detail in the above section (2).

Further, still another embodiment of the present invention is a recording medium that stores the program.

### [Examples]

In this example, melting temperature of DNA in a well is measured using a fluorescent-labeled probe, and a result of discriminating between BRAF gene and its pseudogene is shown.

It is known that the BRAF gene is present on chromosome 7, which is an autosome, and its pseudogene BRAFP1 is present on an X chromosome. The specimen used in this example is genomic DNA of HCT116, which is a human colon cancer-derived cell line, and since it is male genomic DNA, the copy number of pseudogenes of BRAF gene should be half the sum of the wild-type and mutant copy numbers.

First, for the BRAF gene, wild-type and V600E mutant genomic DNAs (final concentration 133 molecules/µL) are prepared. A forward primer (final concentration 0.25 µM), a reverse primer (final concentration 2.0 µM), a fluorescent-labeled probe corresponding to the wild-type (final concentration 0.5 µM), and 1 × master mix (including DNA polymerase and dNTP), which were required for PCR, were added to prepare a PCR reaction solution. At this time, the primer pair was added so that the concentration of the primer pair was asymmetric so that the complementary DNA strand of the fluorescent-labeled probe was excessively amplified.

Sequences of primers and probes are as follows. All of the fluorescent-labeled probes have complementary sequences near both ends, and are designed so that they form a double strand in the molecule. In addition, CAL Fluor Red 610 as a fluorescent dye is bound to the 5' end, and BHQ-2 as a quencher is bound to the 3' end.
Forward primer: 5'-CATGAAGACCTCACAGTAAAAATAGGTGAT-3' (SEQ ID NO: 1)
Reverse primer: 5'-TGGGACCCACTCCATCGA-3' (SEQ ID NO: 2)
Fluorescent-labeled probe corresponding to BRAF gene wild-type: 5'-GGTCTAGCTACAGTGAAATC-3' (SEQ ID NO: 3)

Thereafter, 15 µL of a PCR reaction solution was added so that one wild-type DNA of BRAF gene, one V600E mutant DNA, one pseudogene DNA, or none of them is contained in each well, and DNA was amplified by PCR.

In the PCR reaction, treatment was performed at 96°C for 10 minutes, followed by 59 cycles of cycles (60°C for 2 minutes, then 98°C for 30 seconds), and finally treatment was performed at 60°C for 2 minutes. After the reaction, a change in the fluorescence intensity of each well was observed while a chip provided with the well was cooled from 85°C to 50°C on a temperature control stage, and melting curves were measured and analyzed.

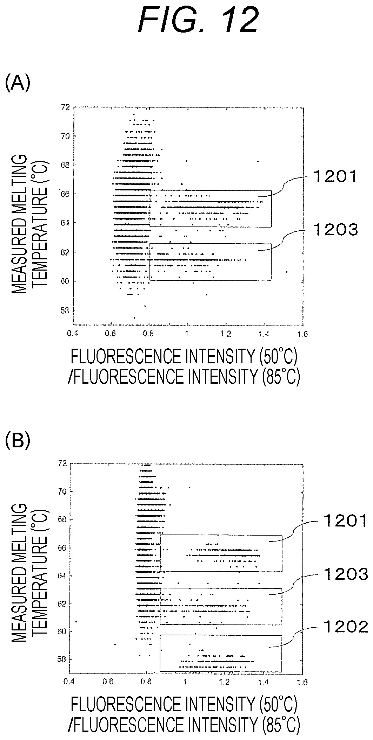

The results are shown in FIG. 12. When two or more fluorescent dyes are used, results as shown in FIG. 12 are obtained for each dye.

FIG. 12A shows results of measuring specimens containing a wild-type BRAF gene and its pseudogene, where the ratio of the fluorescence intensity at 50°C to the fluorescence intensity at 85°C is plotted on the horizontal axis, and the measured melting temperature is plotted on the vertical axis. The results are divided into two distributions depending on the difference in measured melting temperature. A population 1201 having a distribution around 65°C (for example, in the range of 64°C to 66°C) corresponds to wells containing wild-type of BRAF gene, and a population 1203 having a distribution around 61°C (for example, in the range of 60°C to 62°C) corresponds to wells containing a pseudogene of BRAF gene.

FIG. 12B shows results of measuring specimens containing wild-type, a V600E mutant, and a pseudogene of BRAF gene. The results are divided into three distributions depending on the difference in measured melting temperature. A population 1201 having a distribution around 65°C (for example, in the range of 64°C to 66°C) corresponds to wells containing wild-type of BRAF gene, a population 1203 having a distribution around 61°C (for example, in the range of 60°C to 62°C) corresponds to wells containing a pseudogene of BRAF gene, and a population 1202 having a distribution around 58°C (for example, in the range of 57°C to 59°C) corresponds to wells containing a V600E mutant of BRAF gene.

Comparing the count numbers of respective populations in FIG. 12, the count number of pseudogenes of BRAF gene was half the count number of wild-type of BRAF gene in FIG. 12A, and the count number of pseudogenes of BRAF gene was half the sum of the count numbers of wild-type and V600E of BRAF gene in FIG. 12B.

As described above, the measurement accuracy can be guaranteed by discriminating and counting each of the target gene or target DNA and the pseudogene using the measured melting temperature for discrimination of the genotype in the digital PCR, and confirming that the target gene or target DNA and the pseudogene match with the correspondence relation of the copy number obtained in advance from the position on the chromosome.

### Reference Signs List

- 301, 302, 311: droplet (partition)
- 303: microchannel
- 304: light source
- 305: fluorescence filter
- 306: photomultiplemeter (imaging device)
- 307: CCD camera (imaging device)
- 308: lens
- 309: dichroic mirror
- 310: droplet detection cartridge
- 312: temperature control stage (temperature adjustment unit)
- 313: cartridge
- 314, 315: well (partition)
- 401: fluorescence measurement unit
- 402: computer
- 403: analysis unit
- 404: database
- 405: memory (melting temperature memory)
- 406: monitor
- 601: DNA
- 602: fluorescent-labeled probe
- 603: fluorescent dye
- 604: quencher
- 605: melting temperature
- 1201: wells containing wild-type
- 1202: wells containing mutant
- 1203: wells containing pseudogene

## Claims

1. A DNA detection method comprising the steps of:
placing a DNA solution in each of a plurality of partitions, wherein the DNA solution may contain a plurality of types of DNAs including a first gene and a pseudogene of the first gene, and the DNA solution contains a fluorescent-labeled probe or a DNA intercalator;
performing PCR in each of the partitions;
changing a temperature of each of the partitions during the PCR or after the PCR and measuring a fluorescence intensity changing with the temperature change for each of the partitions;
calculating a melting temperature of a double strand DNA placed in the partition, for each of the partitions, based on a change in the fluorescence intensity accompanying the temperature change;
discriminating the type of DNA for each of the partitions based on the melting temperature and counting the number of partitions for each type of the DNA;
outputting the number of partitions counted for each type of the DNA; and
discriminating the first gene and the pseudogene based on the melting temperature and the number of the counted partitions.

2. The DNA detection method according to claim 1, wherein the fluorescent-labeled probe includes a first fluorescent-labeled probe having a sequence corresponding to the first gene.

3. The DNA detection method according to claim 2, wherein the fluorescent-labeled probe includes a second fluorescent-labeled probe having a sequence corresponding to the pseudogene.

4. The DNA detection method according to claim 1, comprising a step of determining whether a ratio of the number of partitions in which the pseudogene is placed to the number of partitions in which the first gene is placed is a value within a range corresponding to 1, a value within a range corresponding to 1/2, a value within a range corresponding to 2, a value within a range corresponding to 0, or a value not within any range.

5. The DNA detection method according to claim 1, comprising a step of displaying the number of the first genes, displaying a percentage of mutant genes in the first genes, or displaying a total number of genes calculated from the number of the first genes or the number of the pseudogenes.

6. The DNA detection method according to claim 1, wherein the DNA solution placed in each of the partitions is subjected to limiting dilution.

7. The DNA detection method according to claim 1, wherein the step of discriminating the type of DNA for each of the partitions based on the melting temperature and counting the number of partitions for each type of the DNA is further performed based on information indicating a predetermined reference melting temperature.

8. The DNA detection method according to claim 1, wherein the partitions are configured as wells arranged in an array or droplets dispersed in oil.

9. The DNA detection method according to claim 1, wherein the melting temperature is calculated as an inflection point of the fluorescence intensity.

10. A DNA detection system comprising:
an imaging device that captures an image of a device capable of arranging a DNA solution in each of a plurality of partitions, wherein the DNA solution may contain a plurality of types of DNAs including a first gene and a pseudogene of the first gene, and the DNA solution contains a fluorescent-labeled probe or a DNA intercalator;
a temperature adjustment unit that changes a temperature of each of the partitions in order to perform PCR in each of the partitions;
a database that stores information indicating a reference melting temperature for each of the first gene and the pseudogene;
an imaging control unit that causes the imaging device to capture an image in order to acquire a fluorescence intensity changing with a temperature change for each of the partitions;
a melting temperature memory that stores information indicating a melting temperature of a double strand DNA placed in the partition, for each of the partitions, wherein the melting temperature is obtained based on a change in the fluorescence intensity accompanying a temperature change in an image captured by the imaging device; and
an analysis unit that counts the number of partitions in which the first gene is placed and the number of partitions in which the pseudogene is placed using the database and the melting temperature memory, wherein the analysis unit outputs the number of partitions counted for each type of the DNA and discriminates the first gene and the pseudogene based on the melting temperature and the number of counted partitions.

11. The DNA detection system according to claim 10, wherein the analysis unit determines whether a ratio of the number of partitions in which the pseudogene is placed to the number of partitions in which the first gene is placed is a value within a range corresponding to 1, a value within a range corresponding to 1/2, a value within a range corresponding to 2, a value within a range corresponding to 0, or a value not within any range.

12. The DNA detection system according to claim 10, wherein the information indicating a reference melting temperature is information indicating a threshold of a temperature range.
